# EUROPEAN PATENT SPECIFICATION

(11) **EP 0 524 989 B1**
(45) Date of publication and mention of the grant of the patent: **16.10.1996**
(21) Application number: 91907396.5
(22) Date of filing: 09.04.1991
(51) Int. Cl.: A61K 9/24, A61K 9/54

(54) **CONTROLLED RELEASE DRUG FORMULATION**
ARZNEIMITTELZUSAMMENSETZUNG MIT KONTROLLIERTER WIRKSTOFFABGABE
COMPOSITION MEDICAMENTEUSE A LIBERATION CONTROLEE

(30) Priority: 17.04.1990 IT 2005490
(43) Date of publication of application: 03.02.1993
(73) Proprietor: EURAND INTERNATIONAL S.P.A., 20060 Pessano Con Bornago, Milan (IT)
(72) Inventor: CALANCHI, Massimo, Maria, I-20052 Monza (IT); ZEMA, Marco, I-22100 Como (IT); GIORGETTI, Enzo, I-20132 Milan (IT); BRUNETTI, Gabriele, I-20129 Milan (IT)
(74) Representative: Wileman, David Francis
(86) International application number: PCT/EP91/00688
(87) International publication number: WO 91/16042

(56) References cited:
- EP-A- 0 148 811
- EP-A- 0 212 745
- EP-A- 0 239 361
- EP-A- 0 277 925
- EP-A- 0 342 522
- WO-A-85/03437
- WO-A-87/01588
- GB-A- 2 066 070
- GB-A- 2 134 785

## Description

The present invention relates to a controlled release drug formulation and method for obtaining a targeted and controlled release of calcitonin or ferritin which must carry out their pharmacological action in the intestine and in particular in the small intestine and/or colon.

USA patent 4,503,030 refers to tablets with osmotic release, consisting of a core containing the drug, covered with a semipermeable and pH-dependent membrane in which a hole is made to put the nucleus in communication with the outside. In the stomach, the tablet remains intact and the release occurs through the hole made in the membrane, while in the intestine the membrane disintegrates completely.

USA patent 4,432,966 describes the preparation of tablets which disintegrate in the colon. This is achieved by coating the tablet core with two layers.

The first is made up of a pH independent polymer and microcrystalline cellulose, the second of a pH dependent polymer. The presence of microcrystalline cellulose together with the pH independent polymer, is essential to assure the disgregation of the tablet in the colon, since the microcrystalline cellulose is digested by specific enzymes and the bacteria present in the colon.

Sustained or delayed release pharmaceutical preparations are disclosed in EP-A-239361, EP-A-148811, EP-A-212745, WO 85/03437, EP-A-277925, EP-A-342522 and WO 87/01588. These citations disclose coated drug cores which are in a form selected from the following: granules, crystals, non pareil or sucrose drug coated core, and spherical granules, all of varying sizes,or a single large tablet. There is no disclosure of a targeted or sustained release formulation where the drug is in the form of a plurality of minitablets.

The present invention has various advantages with respect to those cited above as it relates to multidose forms instead of monodose forms and minitablets instead of microcapsules or the like.

It is known that multidose forms spread in a wide area of the gastro-intestinal tract avoiding and reducing problems of irritation of the mucosa due to a high concentration of the drug, and improving absorption of the same drug.

Moreover in the present invention while the pH dependent membrane dissolves when the coated particles reach the proper pH in the intestine, the pH independent membrane remains intact in order to delay the dissolution of the drug, in a time which can vary from about 30 minutes to about 8 hours, and consequently proLong its action along the small intestine and/or the colon.

According to the present invention there is provided a targeted drug release formulation for delivery of a drug selected from calcitonin and ferritin to the small intestine and/or colon of a mammal consisting essentially of a plurality of minitablets, each said minitablet having a particle size of less than about 5mm and consisting essentially of a core of calcitonin or ferritin surrounded by two membranes, one membrane consisting essentially of a pH dependent polymer which is substantially soluble at a pH greater than about 5.0, selected from anionic copolymers based on methacrylic acid and methacrylic acid methyl or ethyl ester, cellulose acetate phthalate, hydroxypropylmethylcellulose phthalate, polyvinyl acetate phthalate, shellac, hydroxypropylmethylcellulose acetate succinate, carboxymethylcellulose, cellulose acetate trimellitate, copolymers of maleic acid and derivatives of phthalic acid and the second of said membranes consisting essentially of one or more polymers selected from copolymers formed from acrylic and methacrylic acid esters with a low content of quaternary ammonium groups, neutral copolymers based on ethyl acrylate and methyl methacrylate and having an average molecular weight of 800,000, ethylcellulose, polyethylene, polysiloxanes and mixtures thereof such that said second membrane is substantially insoluble in but permeable to gastrointestinal fluids. Said formulation may be further characterised by the release of no more than about 10% of drug in the stomach (or at a pH lower than about 4.5) with a dissolution rate such that over a period of about 30 minutes to about 8 hours substantially all of the remaining drug is released in the intestine and/or colon (or at a pH of 5 or more in simulated gastrointestinal medium). The release may be further characterised by the release of no more than about 11% drug after 3 hours and no more than about 75% drug after 6 hours in simulated gastrointestinal juices. Another embodiment is where the dissolution is to occur in the small intestine it is preferred that substantially all of the release (90%) occurs between 1 hour and 1 1/2 hours at pH 6.8, with no more than 10% release occuring in the stomach.

Typical pH values are 1 to 3.5 for the stomach, 5 to 6, for the duodenum, 6 to 7 for the jejunum and pH 7 to 8 for the ileum.

The present invention is suitable for calcitonin and ferritin which are destroyed by gastric juices or inactivated by enzymes such as for example pancreatic and bacterial proteases of the ileum.

For some diseases of the intestine, and in particular of the colon, it is important that the drugs are transported intact to the place in which they will carry out their pharmacological action.

This is achieved by coating them with a membrane with pH-dependent solubility, and more particularly with a membrane which is soluble at a pH greater than 5.0, so that it remains intact in the stomach and first part of the intestine while it dissolves when a pH of greater than 5.0 is reached in the intestine, thus releasing the drug. But for various drugs it is also important that the contact with the mucosa, or their absorption, occurs along all the colon, and therefore it is necessary to delay the release so that the effect is prolonged in time and does not occur only in the initial tract, as happens when the drug is covered with the pH dependent membrane only.

It has now been discovered that by applying separately a membrane with pH dependent solubility and a membrane which is insoluble but permeable to gastrointestinal fluids, the dissolution of the drug can be delayed; it is released slowly and can thus carry out its action along the small intestine or the colon, or both. In fact if the drug is coated by a Eudragit S membrane, (which dissolves at a pH higher than 7) there is a very low release in buffered solutions up to pH 6.2 (first 3 hours), but when the pH increases to 7.2 a rapid dissolution of the drug occurs.

Only by applying a second membrane of ethylcellulose, which is insoluble in the juices but permeable to same, on to the Eudragit membrane, is one able to delay the release of the drug and to prolong the effect for another 3 hours.

The same result is obtained if the delaying membrane is applied before the pH dependent membrane, while if the two types of polymers constituting the membrane are mixed, the delayed effect is not obtained. Instead there is a release very similar to that obtained by applying only the pH dependent polymer.

Moreover, the drug can be targeted to the small intestine or to the colon by making a suitable choice of the pH dependent polymer. For example if we use as pH dependent membrane Eudragit L30D, that is soluble at pH higher than 5.5, it is dissolved in the duodenum, then the pH independent membrane delays the release of the drug along the jejunum or the jejunum plus ileum or the small intestine plus colon depending from the permeability and the amount of pH independent membrane applied on the core. On the contrary, if we use as pH dependent membrane Eudragit S, that is soluble at pH higher than 7, it is dissolved only in the ileum, therefore the pH independent membrane will prolong the release of the drug only along the colon.

The original characteristic of the present invention consists therefore of the consecutive application, in any order, of a membrane soluble at a given pH and an insoluble but permeable membrane.

Thus a release of the drug targeted at a certain tract of the intestine and a prolonging of this release is obtained in such a way as to render it effective along the whole of the remaining intestinal tract.

### Description of the Process for Coating with the First Membrane

The present invention is applied to multidose forms, that is drugs in the form of or tablets of very small dimensions, (also called minitablets) which are coated as described later. These coated drugs are then formulated in capsules, monodose sachets, in rapidly disgregating tablets or in other pharmaceutical forms suitable for oral administration.

The sizes of the single units of the multidose forms, that is of the minitablets, may vary from 0.1 to 3.5 mm but must not exceed 5 mm.

In fact the smaller the single units are, the wider the distribution in the gastrointestinal tract, and furthermore, while the units greater than 5 mm are retained in a full stomach, units smaller than 5 mm pass through the stomach much more rapidly and in a similar way to liquids.

This phenomenon is described in the article by S.S. Davis "The Design and Evaluation of the Controlled Release Systems for the Gastrointestinal Tract" published in the "Journal of Controlled Release", 2(1985)27-38.

Since the drugs are often in fine powder form, these are generally granulated, using known dry or wet techniques (compacting), to obtain the desired particle size.

However it should be considered that the description which follows, that is referring to drugs in granular form is also valid for minitablets.

The granulated drug is placed in a UNI Glatt fluid bed container equipped with the Wurster insert and is coated, by spraying through atomizer, with a pH dependent polymer, dissolved in an organic solvent, or in a mixture of organic solvents, or in a mixture of organic solvents and water, or in solution, dispersion or aqueous emulsion.

It is also convenient to add plasticisers. Among the types of polymers constituting the pH dependent membrane the following are cited as an illustrative but not limiting example: anionic co-polymers of methacrylic acid and methacrylic acid methyl or ethyl ester (e.g Eudragit L, S, L30D, L100-55), cellulose acetate phthalate, hydroxypropylmethylcellulose phthalate,(e.g HP 50) polyvinyl acetate phthalate, shellac, hydroxypropylmethyl-cellulose acetate succinate, (e.g AS-L) carboxymethylcellulose, cellulose acetate trimellitate, copolymers of maleic acid and phthalic acid derivatives.

Cited among the plasticisers are polyethylene glycol, dibutyl phthalate, diethyl phthalate, citric acid esters and among the adjuvants: talc, silicon dioxide, titanium dioxide, magnesium stearate, again as an illustrative but not limiting example.

The coated granules are dried, e.g. with hot air (about 50 degrees C) for about 30 minutes.

### Description of the Second Membrane Coating Process

These granules coated with pH dependent membrane are then coated with a second pH independent membrane using analogous techniques. Also in the case one can use organic or aqueous solutions or aqueous dispersions/emulsions and it is convenient to add plasticisers and adjuvants of the above indicated type.

The following are cited among the types of polymers constituting the pH independent membrane as an illustrative but not limited example: copolymers of acrylic and methacrylic acid esters with a low content of quaternary ammonium groups, neutral copolymers based on ethyl acrylate and methyl methacrylate and having an average molecular weight of 800,000 (Eudragit RS/RL/NE) ethylcellulose, polyethylene, polysiloxane, alone or mixed with each other or with water-soluble pH independent polymers such as: hydroxypropylmethylcellulose, hydroxypropylcellulose, hydroxyethylcellulose, methylcellulose, polyvinylpyrrolidone.

The granules coated with the membrane are dried, eg with hot air (about 50 degrees C) for about 30 minutes.

As previously mentioned the coating with the two membranes can also be done in the opposite order to that described.

Example 4 illustrates the preparation of coated minitablets according to the invention; reference Examples 1-3 are not part of the claimed invention:

### Example 1 (reference Example)

800g of mebeverine hydrochloride granulated with hydroxypropylmethylcellulose and with a particle size between 710 and 1300µ was put in the UNI Glatt fluid bed container equipped with the Wurster insert.

This granulate was coated with a first membrane of Eudragit S, by spraying a suspension with the following composition with the atomiser: 468g of methylene chloride, 156g of isopropyl alcohol, 55.6g of Eudragit S, 5.5g of dibutyl phthalate and 28g of talc.

The coated granules were dried in hot air (about 50 degrees C) for 30 minutes and then the release was determined with the USP apparatus 2 (paddle), utilising the following sequence of artificial juices, 2 hours in 0.1N HCl, 1 hour in pH 6.2 buffer and the following hours in pH 7.2 buffer.

The following results were obtained:

| | | | | | |
|---|---|---|---|---|---|
| Time (hours) | 1 | 2 | 3 | 4 | 5 |
| Release (%) | 8 | 14 | 16 | 70 | 97 |

Then a second membrane of ethylcellulose was applied to 700g of these Eudragit S coated granules by spraying the following solution 199g of methylene chloride, 44g of ethyl alcohol, 4.3g of ethylcellulose, 8.6g of hydroxypropylmethylcellulose and 1.5g of diacetylated monoglycerides and finally drying with air at 50 degrees C for about 30 minutes. The granules coated with the two membranes were analysed again as described above and the following results were obtained:

| | | | | | | | |
|---|---|---|---|---|---|---|---|
| Time (hours) | 1 | 2 | 3 | 4 | 5 | 6 | 8 |
| Release (%) | 5 | 10 | 11 | 28 | 49 | 73 | 95 |

### Example 2 (reference Example)

700g of calcitonin granulated with lactose and hydroxypropylmethylcellulose as binder with a particle size between 710 and 1300µ was put in the UNI Glatt container equipped with Wurster insert.

These granules were coated with a first membrane of ethylcellulose/hydroxypropylmethylcellulose, by spraying solution with the following composition with the atomiser: 200g of methylene chloride, 45g of ethyl alcohol, 6.4g of ethylcellulose, 6.4g of hydroxypropylmethylcellulose, and 1.4g of diacetylated monoglycerides.

The coated granules were dried with hot air (about 50 degrees C) for 30 minutes and then its release was determined with the USP apparatus 2 (paddle), utilising the following sequence of artificial juices: 2 hours in HCl 0.1N, 1 hour in pH 6.2 buffer and the following hours in pH 7.2 buffer.

The following results were obtained:

| | | | | | |
|---|---|---|---|---|---|
| Time (hours) | 1 | 2 | 3 | 4 | 5 |
| Release (%) | 27 | 51 | 69 | 83 | 99 |

Then a second Eudragit S membrane was applied to these coated granules, by spraying the following suspension: 134 g of methylene chloride, 65g of isopropyl alcohol, 23g of Eudragit S, 2.3g of dibutyl phthalate and 11.5g of talc and finally drying with air at 50 degrees C for about 30 minutes.

The granules coated with the two membranes were analysed again as described above and the following results were obtained:

| | | | | | | | |
|---|---|---|---|---|---|---|---|
| Time (hours) | 1 | 2 | 3 | 4 | 5 | 6 | 8 |
| Release (½) | 2 | 3 | 7 | 26 | 58 | 72 | 98 |

### Example 3 (reference Example)

800g of mebeverine hydrochloride granulated with hydroxypropylmethylcellulose with a particle size between 710 and 1300 um was put in the UNI Glatt fluid bed container equipped with Wurster insert.

These granules were coated with ethylcellulose hydroxypropylmethylcellulose / Eudragit S membrane, by spraying a suspension with the following composition with an atomiser: 836g of methylene chloride, 418g of isopropyl alcohol, 5.8g of ethylcellulose, 11.8g of hydroxypropylmethylcellulose, 58.7g of Eudragit S, 3.7g of dibutyl phthalate and 29g of talc.

The coated granules were dried with hot air (about 45 degrees C) for 30 minutes and then the release was determined with the USP apparatus 2 (paddle) using the following sequence of artificial juices: 2 hours in 0.1 N HCl, 1 hour in pH 6.2 buffer and the following hours in pH 7.2 buffer.

The following results were obtained:

| | | | | | | |
|---|---|---|---|---|---|---|
| Time (hours) | 1 | 2 | 3 | 4 | 5 | 6 |
| Releases (%) | 4 | 7 | 10 | 58 | 90 | 100 |

### Example 4

Very small tablets of Ferritin, a protein that is destroyed by the gastric juices, were prepared. They had a diameter of 2.0 mm and a thickness of 2.2 mm, and the following composition:

| | |
|---|---|
| Ferritin | 80.0% |
| Magnesium Stearate | 1.5% |
| Silicon dioxide | 1.0% |
| Microcrystalline cellulose | 13.5% |
| Hydrogenated castor oil | 1.0% |
| Sodium Crosscarmellose | 3.0% |

2.2 kg of these Ferritin minitablets were put in the Versaglatt container (fluid bed coater) equipped with the Wurster insert.

They were coated first with a membrane of ethylcellulose aqueous dispersion (Aquacoat ^{R}/FMC) and hydroxypropyl methylcellulose, by spraying a suspension with the following composition:

| | |
|---|---|
| - Aquacoat ^{R}(30% aqueous dispersion w/w) | 24.30 g |
| - Methocel E% (10% aqueous solution w/w) | 290.00 g |
| - Dibutylsebacate | 1.48 g |
| - Talc | 7.26 g |
| (total solid content of the suspension: 14% w/w) | |

The coated minitablets were dried in hot air (about 60°C) for 30 minutes and then the release was determined with the USP apparatus 2 (paddle) in pH 6.8 buffer. The following results were obtained:

| | | | |
|---|---|---|---|
| Time (minutes) | 30 | 60 | 90 |
| Release (%) | 27 | 76 | 100 |

Then an aqueous acrylic resin dispersion (Eudragit L30D) was applied on these coated minitablets.

It had the following composition:

| | |
|---|---|
| - Eudragit L30D (30% aqueous dispersion w/w) | 273 g |
| - Talc | 33 g |
| - Triethylcitrate | 8 g |
| - Deionized water | 286 g |
| (total solid content: 20% w/w) | |

The double coated minitablets were dried with air at 50°C for about 30 minutes and analysed with the USP apparatus 2 (paddle) using the following sequence of artificial juices: 60′ in 0.1 N HCl, 60′ at pH 4.5 buffer and then at pH 6.8 buffer.

The following results were obtained:

| | | | | | |
|---|---|---|---|---|---|
| Time (minutes) | 60 | 120 | 150 | 180 | 210 |
| Release (%) | 2 | 10 | 38 | 85 | 100 |

This example illustrates a formulation targetting the small intestine where substantially all of the release occurs at pH 6.8 over a period of 1 to 1½ hours.

## Claims (Claims for the following Contracting State(s): AT, BE, CH, DE, DK, FR, GB, IT, LI, LU, NL, SE)

1. A targeted drug release formulation for delivery of a drug selected from calcitonin and ferritin to the small intestine and/or colon of a mammal consisting essentially of a plurality of minitablets each said minitablet having a particle size of less than about 5 mm and consisting essentially of a core of calcitonin or ferritin surrounded by two membranes, one membrane consisting essentially of a pH dependent polymer which is substantially soluble at a pH greater than about 5.0 so that it remains intact in the stomach and final part of the intestine, selected from anionic copolymers based on methacrylic acid and methacrylic acid methyl or ethyl ester, cellulose acetate phthalate, hydroxypropylmethylcellulose phthalate, polyvinyl acetate phthalate, shellac, hydroxypropylmethylcellulose acetate succinate, carboxymethylcellulose, cellulose acetate trimellitate, copolymers of maleic acid and derivatives of phthalic acid and the second of said membranes consisting essentially of one or more polymers selected from copolymers formed from acrylic and methacrylic acid esters with a low content of quaternary ammonium groups, neutral copolymers based on ethyl acrylate and methyl methacrylate and having an average molecular weight of 800,000, ethylcellulose, polyethylene, polysiloxanes and mixtures thereof such that said second membrane is substantially insoluble in but permeable to gastrointestinal fluids so that it remains intact to delay the dissolution of the drug in a time which can vary from 30 minutes to 8 hours.

2. A targeted drug release formulation as claimed in Claim 1 wherein said formulation is characterised by the release of no more than about 10% drug into the stomach with substantially all of the remainder of the drug being released in the small intestine and/or colon in a period from ½ hour to 8 hours.

3. A targeted drug formulation as claimed in Claim 1 wherein said formulation is characterised by the release of no more than about 10% of the drug at pH lower than 4.5 with substantially all of the remainder of the drug being released at a pH of 5.0 or greater in a period of from ½ hour to 8 hours in simulated gastrointestinal media.

4. The formulation of any one of Claims 1 to 3 in which the membrane containing the pH dependent polymer is interior to the other membrane.

5. The formulation of any one of Claims 1 to 3 in which the membrane containing the pH dependent polymer is exterior to the other membrane.

6. The formulation of any one of Claims 1 to 5 further comprising a pH independent water soluble polymer selected from hydroxypropylmethylcellulose, hydroxypropylcellulose, hydroxyethylcellulose, methylcellulose, polyvinylpyrrolidone and mixtures thereof.

7. The formulation of any one of the preceding claims further comprising a plasticizer in at least one of the membranes.

8. The formulation of Claims 1 to 5 wherein said substantially insoluble membrane is selected from ethyl cellulose and mixtures of ethylcellulose with hydroxypropylmethylcellulose in a ratio of 1:5 to 5:1.

9. The formulation of any one of Claims 1 to 8 in which the minitablets have a particle size between 0.1 mm and 5.0 mm

10. A pharmaceutical dosage form comprising the formulation of anyone of the preceding claims further formulated in capsules or tablets.

11. A pharmaceutical dosage form as claimed in any one of the preceding claims which is characterised by the release of no more than about 11% after 3 hours and no more than about 75% after 6 hours in simulated gastrointestinal juices.

12. A pharmaceutical dosage form as claimed in any one of Claims 1 - 9 which is characterised by the release of about 90% of the drug in a period of 1 to 1 1/2 hours at pH 6.8.

13. A method for preparing a targeted drug release formulation as defined in anyone of the preceding Claims comprising the steps of
A) preparing a plurality of minitablets having a particle size no greater than about 5 mm,
B) coating said minitablets with two separate and distinctly characterised membrane layers each as defined in Claim 1,
C) if desired formulating said coated minitablet into drug dose oral delivery forms selected from capsules or tablets.

## Claims (Claims for the following Contracting State(s): ES, GR)

1. A process for preparing a targeted drug release formulation for delivery of a drug selected from calcitonin and ferritin to the small intestine and/or colon of a mammal which comprises the steps of
A) preparing a plurality of minitablets each said minitablet having a particle size of less than about 5mm and consisting essentially of a core of calcitonin or ferritin
B) coating said minitablets with two separate and distinctly characterised membranes, one membrane consisting essentially of a pH dependent polymer which is substantially soluble at a pH greater than about 5.0 so that it remains intact in the stomach and final part of the intestine, selected from anionic copolymers based on methacrylic acid and methacrylic acid methyl or ethyl ester, cellulose acetate phthalate, hydroxypropylmethylcellulose phthalate, polyvinyl acetate phthalate, shellac, hydroxypropylmethylcellulose acetate succinate, carboxymethylcellulose, cellulose acetate trimellitate, copolymers of maleic acid and derivatives of phthalic acid and the second of said membranes consisting essentially of one or more polymers selected from copolymers formed from acrylic and methacrylic acid esters with a low content of quaternary ammonium groups, neutral copolymers based on ethyl acrylate and methyl methacrylate and having an average molecular weight of 800,000, ethylcellulose, polyethylene, polysiloxanes and mixtures thereof such that said second membrane is substantially insoluble in but permeable to gastrointestinal fluids so that it remains intact to delay the dissolution of the drug in a time which can vary from 30 minutes to 8 hours, and
C) if desired formulating said coated minitablets into drug dose oral delivering forms selected from capsules or tablets.

2. A process for preparing a targeted drug release formulation as claimed in Claim 1 wherein said formulation is characterised by the release of no more than about 10% drug into the stomach with substantially all of the remainder of the drug being released in the small intestine and/or colon in a period from 1/2 hour to 8 hours.

3. A process for preparing a targeted drug formulation as claimed in Claim 1 wherein said formulation is characterised by the release of no more than about 10% of the drug at pH lower than 4.5 with substantially all of the remainder of the drug being released at a pH of 5.0 or greater in a period of from about 1/2 hour to 8 hours in simulated gastrointestinal media.

4. A process for preparing a formulation as claimed in any one of Claims 1 to 3 in which the membrane containing the pH dependent polymer is interior to the other membrane.

5. A process for preparing a formulation as claimed in any one of Claims 1 to 3 in which the membrane containing the pH dependent polymer is exterior to the other membrane.

6. A process for preparing a formulation as claimed in any one of Claims 1 to 5 further comprising a pH independent water soluble polymer selected from hydroxypropylmethylcellulose, hydroxypropylcellulose, hydroxyethylcellulose, methylcellulose, polyvinylpyrrolidone and mixtures thereof.

7. A process for preparing a formulation as claimed in any one of the preceding claims further comprising a plasticizer in at least one of the membranes.

8. A process for preparing a formulation as claimed in Claims 1 to 5 wherein said substantially insoluble membrane is selected from ethyl cellulose and mixtures of ethylcellulose with hydroxypropylmethylcellulose in a ratio of 1:5 to 5:1.

9. A process for preparing a formulation as claimed in any one of Claims 1 to 8 in which the minitablets have a particle size between 0.1 mm and 5.0 mm

10. A process for preparing a targeted drug release formulation according to the preceding claims where the coated minitablets are further formulated into capsules or tablets.

11. A process as claimed in any one of the preceding claims in which the formulation prepared is characterised by the release of no more than about 11% after 3 hours and no more than about 75% after 6 hours in simulated gastrointestinal juices.

12. A process as claimed in any one of Claims 1 - 9 in which the formulation prepared is characterised by the release of about 90% of the drug in a period of 1 to 1 1/2 hours at pH 6.8.

## Patentansprüche (Patentansprüche für folgende(n) Vertragsstaat(en): AT, BE, CH, DE, DK, FR, GB, IT, LI, LU, NL, SE)

1. Formulierung mit gezielter Arzneimittelfreisetzung zur Abgabe eines Arzneimittels, ausgewählt aus Calcitonin und Ferritin, im Dünndarm und/oder Colon eines Säugers, welche im wesentlichen aus einer Vielzahl von Minitabletten besteht, wobei jede Minitablette eine Teilchengröße von weniger als etwa 5 mm aufweist und im wesentlichen aus einem Calcitonin- oder Ferritin-Kern besteht, der von zwei Membranen umgeben ist, wobei eine Membran im wesentlichen aus einem pH-abhängigen Polymer besteht, das bei einem pH von mehr als etwa 5,0 im wesentlichen löslich ist, so daß es im Magen und im Enddarmteil intakt bleibt, ausgewählt aus anionischen Copolymeren auf der Basis von Methacrylsäure und Methacrylsäuremethyl- oder -ethylester, Celluloseacetatphthalat, Hydroxypropylmethylcellulosephthalat, Polyvinylacetatphthalat, Schellack, Hydroxypropylmethylcelluloseacetatsuccinat, Carboxymethylcellulose, Celluloseacetattrimellitat, Copolymeren von Maleinsäure und Derivaten von Phthalsäure, und die zweite der Membranen im wesentlichen aus einem oder mehreren Polymeren besteht, ausgewählt aus Copolymeren, die gebildet werden aus Acrylsäure- und Methacrylsäureestern mit einen geringen Gehalt an quaternären Ammonium-Gruppen, neutralen Copolymeren auf der Basis von Ethylacrylat und Methylmethacrylat und mit einer mittleren Molmasse von 800 000, Ethylcellulose, Polyethylen, Polysiloxanen und Mischungen hievon, so daß die zweite Membran in Gastrointestinalflüssigkeiten im wesentlichen unlöslich, jedoch für diese durchlässig ist, so daß sie intakt bleibt, um das Lösen des Arzneimittels für eine Zeit zu verzögern, die von 30 Minuten bis 8 Stunden variieren kann.

2. Formulierung mit gezielter Arzneimittelfreisetzung nach Anspruch 1, wobei die Formulierung gekennzeichnet ist durch die Freisetzung von nicht mehr als etwa 10 % Arzneimittel im Magen und durch die Freisetzung des gesamten Rests des Arzneimittels im Dünndarm und/oder Colon in einem Zeitraum von ½ Stunde bis 8 Stunden.

3. Formulierung mit gezielter Arzneimittelfreisetzung nach Anspruch 1, wobei die Formulierung gekennzeichnet ist durch die Freisetzung von nicht mehr als etwa 10 % Arzneimittel bei einem pH von weniger als 4,5 und durch die Freisetzung des gesamten Rests des Arzneimittels bei einem pH von 5,0 oder mehr in einem Zeitraum von ½ Stunde bis 8 Stunden in simulierten Gastrointestinalmedien.

4. Formulierung nach einem der Ansprüche 1 bis 3, wobei die das pH-abhängige Polymer enthaltende Membran innerhalb der anderen Membran liegt.

5. Formulierung nach einem der Ansprüche 1 bis 3, wobei die das pH-abhängige Polymer enthaltende Membran außerhalb der anderen Membran liegt.

6. Formulierung nach einem der Ansprüche 1 bis 5, welche ferner ein pH-unabhängiges wasserlösliches Polymer, ausgewählt aus Hydroxypropylmethylcellulose, Hydroxypropylcellulose, Hydroxyethylcellulose, Methylcellulose, Polyvinylpyrrolidon und Mischungen hievon, enthält.

7. Formulierung nach einem der vorhergehenden Ansprüche, welche ferner einen Weichmacher zumindest in einer der Membranen umfaßt.

8. Formulierung nach einem der Ansprüche 1 bis 5, wobei die im wesentlichen unlösliche Membran ausgewählt ist aus Ethylcellulose und Mischungen von Ethylcellulose mit Hydroxypropylmethylcellulose in einem Verhältnis von 1:5 bis 5:1.

9. Formulierung nach einem der Ansprüche 1 bis 8, bei welcher die Minitabletten eine Teilchengröße zwischen 0,1 mm und 5,0 mm aufweisen.

10. Pharmazeutische Dosierungsform, welche die Formulierung nach einem der vorhergehenden Ansprüche, weiter formuliert in Kapseln oder Tabletten, umfaßt.

11. Pharmazeutische Dosierungsform nach einem der vorhergehenden Ansprüche, welche gekennzeichnet ist durch die Freisetzung von nicht mehr als etwa 11 % nach 3 Stunden und nicht mehr als etwa 75 % nach 6 Stunden in simulierten Gastrointestinalsäften.

12. Pharmazeutische Dosierungsform nach einem der Ansprüche 1 bis 9, welche gekennzeichnet ist durch die Freisetzung von etwa 90 % des Arzneimittels in einem Zeitraum von 1 bis 1 ½ Stunden bei pH 6,8.

13. Verfahren zur Herstellung einer Formulierung mit gezielter Arzneimittelfreisetzung, wie in einem der vorhergehenden Ansprüche definiert, welches die Schritte umfaßt:
A) Herstellen einer Vielzahl von Minitabletten mit einer Teilchengröße von nicht mehr als etwa 5 mm,
B) Überziehen der Minitabletten mit zwei getrennten und eindeutig charakterisierten Membranschichten, jeweils wie in Anspruch 1 definiert,
C) gewünschtenfalls Formulieren der überzogenen Minitablette in Arzneimitteldosierungsformen zur oralen Abgabe, ausgewählt aus Kapseln oder Tabletten.

## Patentansprüche (Patentansprüche für folgende(n) Vertragsstaat(en): ES, GR)

1. Verfahren zur Herstellung einer Formulierung mit gezielter Arzneimittelfreisetzung zur Abgabe eines Arzneimittels, ausgewählt aus Calcitonin und Ferritin, im Dünndarm und/oder Colon eines Säugers, welches die Schritte umfaßt:
A) Herstellen einer Vielzahl von Minitabletten, wobei jede Minitablette eine Teilchengröße von weniger als etwa 5 mm aufweist und im wesentlichen aus einem Calcitonin- oder Ferritin-Kern besteht,
B) Überziehen der Minitabletten mit zwei getrennten und eindeutig charakterisierten Membranen, wobei eine Membran im wesentlichen aus einem pH-abhängigen Polymer besteht, das bei einem pH von mehr als etwa 5,0 im wesentlichen löslich ist, so daß es im Magen und im Enddarmtell intakt bleibt, ausgewählt aus anionischen Copolymeren auf der Basis von Methacrylsäure und Methacrylsäuremethyl- oder -ethylester, Celluloseacetatphthalat, Hydroxypropylmethylcellulosephthalat, Polyvinylacetatphthalat, Schellack, Hydroxypropylmethylcelluloseacetatsuccinat, Carboxymethylcellulose, Celluloseacetattrimellitat, Copolymeren von Maleinsäure und Derivaten von Phthalsäure, und die zweite der Membranen im wesentlichen aus einem oder mehreren Polymeren besteht, ausgewählt aus Copolymeren, die gebildet werden aus Acrylsäure- und Methacrylsäureestern mit einen geringen Gehalt an quaternären Ammonium-Gruppen, neutralen Copolymeren auf der Basis von Ethylacrylat und Methylmethacrylat und mit einer mittleren Molmasse von 800 000, Ethylcellulose, Polyethylen, Polysiloxanen und Mischungen hievon, so daß die zweite Membran in Gastrointestinalflüssigkeiten im wesentlichen unlöslich, jedoch für diese durchlässig ist, so daß sie intakt bleibt, um das Lösen des Arzneimittels für eine Zeit zu verzögern, die von 30 Minuten bis 8 Stunden variieren kann, und
C) gewünschtenfalls Formulieren der überzogenen Minitablette in Arzneimitteldosierungsformen zur oralen Abgabe, ausgewählt aus Kapseln oder Tabletten.

2. Verfahren zur Herstellung einer Formulierung mit gezielter Arzneimittelfreisetzung nach Anspruch 1, wobei die Formulierung gekennzeichnet ist durch die Freisetzung von nicht mehr als etwa 10 % Arzneimittel im Magen und durch die Freisetzung des gesamten Rests des Arzneimittels im Dünndarm und/oder Colon in einem Zeitraum von ½ Stunde bis 8 Stunden.

3. Verfahren zur Herstellung einer Formulierung mit gezielter Arzneimittelfreisetzung nach Anspruch 1, wobei die Formulierung gekennzeichnet ist durch die Freisetzung von nicht mehr als etwa 10 % Arzneimittel bei einem pH von weniger als 4,5 und durch die Freisetzung des gesamten Rests des Arzneimittels bei einem pH von 5,0 oder mehr in einem Zeitraum von etwa ½ Stunde bis 8 Stunden in simulierten Gastrointestinalsäften.

4. Verfahren zur Herstellung einer Formulierung nach einem der Ansprüche 1 bis 3, wobei die das pH-abhängige Polymer enthaltende Membran innerhalb der anderen Membran liegt.

5. Verfahren zur Herstellung einer Formulierung nach einem der Ansprüche 1 bis 3, bei welcher die das pH-abhängige Polymer enthaltende Membran außerhalb der anderen Membran liegt.

6. Verfahren zur Herstellung einer Formulierung nach einem der Ansprüche 1 bis 5, welche ferner ein pH-unabhängiges wasserlösliches Polymer, ausgewählt aus Hydroxypropylmethylcellulose, Hydroxypropylcellulose, Hydroxyethylcellulose, Methylcellulose, Polyvinylpyrrolidon und Mischungen hievon, enthält.

7. Verfahren zur Herstellung einer Formulierung nach einem der vorhergehenden Ansprüche, die ferner einen Weichmacher zumindest in einer der Membranen umfaßt.

8. Verfahren zur Herstellung einer Formulierung nach einem der Ansprüche 1 bis 5, wobei die im wesentlichen unlösliche Membran ausgewählt wird aus Ethylcellulose und Mischungen von Ethylcellulose mit Hydroxypropylmethylcellulose in einem Verhältnis von 1:5 bis 5:1.

9. Verfahren zur Herstellung einer Formulierung nach einem der Ansprüche 1 bis 8, wobei die Minitabletten eine Teilchengröße zwischen 0,1 mm und 5,0 mm aufweisen.

10. Verfahren zur Herstellung einer Formulierung mit gezielter Arzneimittelfreisetzung nach einem der vorhergehenden Ansprüche, wobei die überzogenenen Minitabletten weiter in Kapseln oder Tabletten formuliert werden.

11. Verfahren nach einem der vorhergehenden Ansprüche, wobei die hergestellte Formulierung gekennzeichnet ist durch die Freisetzung von nicht mehr als etwa 11 % nach 3 Stunden und nicht mehr als etwa 75 % nach 6 Stunden in simulierten Gastrointestinalsäften.

12. Verfahren nach einem der Ansprüche 1 bis 9, wobei die hergestellte Formulierung gekennzeichnet ist durch die Freisetzung von etwa 90 % des Arzneimittels in einem Zeitraum von 1 bis 1 ½ Stunden bei pH 6,8.

## Revendications (Revendications pour l'(les) Etat(s) contractant(s) suivant(s): AT, BE, CH, DE, DK, FR, GB, IT, LI, LU, NL, SE)

1. Composition médicamenteuse à libération ciblée pour administrer un médicament choisi parmi la calcitonine et la ferritine à l'intestin grêle et/ou au côlon d'un mammifère, consistant essentiellement en une série de mini-comprimés, chaque mini-comprimé ayant une taille de particule inférieure à environ 5 mm et consistant essentiellement en un coeur de calcitonine ou de ferritine, piégé par deux membranes, l'une des membranes consistant essentiellement en un polymère dépendant du pH qui est sensiblement soluble à un pH supérieur à environ 5,0 de sorte qu'il reste intact dans l'estomac et la partie finale de l'intestin, choisi parmi les copolymères anioniques à base d'acide méthacrylique et d'ester méthylique ou éthylique d'acide méthacrylique, d'acétophtalate de cellulose, de phtalate d'hydroxypropylméthylcellulose, d'acétophtalate de polyvinyle, de gomme-laque, d'acétosuccinate d'hydroxypropylméthylcellulose, de carboxyméthylcellulose, d'acétotriméllitate de cellulose, des copolymères d'acide maléique et des dérivés d'acide phtalique et la deuxième des membranes consistant essentiellement en un ou plusieurs polymères choisis parmi les copolymères formés à partir des esters des acides acrylique et méthacrylique avec une faible teneur en groupes ammonium quaternaire, les copolymères neutres à base d'acrylate d'éthyle et de méthacrylate de méthyle et ayant un poids moléculaire moyen de 800 000, l'éthylcellulose, le polyéthylène, les polysiloxanes et leurs mélanges de sorte que la deuxième membrane soit sensiblement insoluble, mais perméable aux fluides gastro-intestinaux, de manière à ce qu'elle reste intacte afin de retarder la dissolution du médicament jusqu'à un moment qui peut varier de 30 minutes à 8 heures.

2. Composition médicamenteuse à libération ciblée suivant la revendication 1, dans laquelle la composition est caractérisée par la libération de pas plus d'environ 10% du médicament dans l'estomac, sensiblement tout le reste du médicament étant libéré dans l'intestin grêle et/ou le côlon, en une période d'1/2 heure à 8 heures.

3. Composition médicamenteuse ciblée suivant la revendication 1, dans laquelle la composition est caractérisée par la libération de pas plus d'environ 10% du médicament à un pH inférieur à 4,5, sensiblement tout le reste du médicament étant libéré à un pH de 5,0 ou davantage, en un période de 1/2 heure à 8 heures dans un milieu gastro-intestinal simulé.

4. Composition suivant l'une quelconque des revendications 1 à 3, dans laquelle la membrane contenant le polymère dépendant du pH est intérieure à l'autre membrane.

5. Composition suivant l'une quelconque des revendications 1 à 3, dans laquelle la membrane contenant le polymère dépendant du pH est extérieure à l'autre membrane.

6. Composition suivant l'une quelconque des revendications 1 à 5, comprenant en outre, un polymère soluble dans l'eau, indépendant du pH, choisi parmi l'hydroxypropylméthylcellulose, l'hydroxypropylcellulose, l'hydroxyéthylcellulose, la méthylcellulose, la polyvinylpyrrolidone et leurs mélanges.

7. Composition suivant l'une quelconque des revendications précédentes, comprenant en outre un plastifiant dans au moins l'une des membranes.

8. Composition suivant les revendications 1 à 5, dans laquelle la membrane sensiblement insoluble est choisie parmi l'éthylcellulose et les mélanges d'éthylcellulose avec l'hydroxypropylméthylcellulose, en un rapport de 1:5 à 5:1.

9. Composition suivant l'une quelconque des revendications 1 à 8, dans laquelle les mini-comprimés ont une taille des particules située entre 0,1 mm et 5,0 mm.

10. Forme de dosage pharmaceutique comprenant la composition suivant l'une quelconque des revendications précédentes, formulée en outre sous forme de capsules ou de comprimés.

11. Forme de dosage pharmaceutique suivant l'une quelconque des revendications précédentes, qui est caractérisée par la libération de pas plus d'environ 11% après 3 heures, et de pas plus d'environ 75% après 6 heures, dans des sucs gastro-intestinaux simulés.

12. Forme de dosage pharmaceutique suivant l'une quelconque des revendications 1 à 9, qui est caractérisée par la libération d'environ 90% du médicament en une période de 1 heure à 1 1/2 heure à pH 6,8.

13. Procédé de préparation d'une composition médicamenteuse à libération ciblée tel que défini dans l'une quelconque des revendications précédentes, comprenant les étapes de :
A) préparation d'une série de mini-comprimés ayant une taille de particule non supérieure à environ 5 mm,
B) enrobage des mini-comprimés au moyen de deux couches de membrane, séparées et caractérisées de manière distincte, chacune définie à la revendication 1, et
C) si souhaité, formulation des mini-comprimés enrobés en des formes dosées médicamenteuses à administration orale, choisies parmi les capsules et les comprimés.

## Revendications (Revendications pour l'(les) Etat(s) contractant(s) suivant(s): ES, GR)

1. Procédé de préparation d'une composition médicamenteuse à libération ciblée pour l'administration d'un médicament choisi parmi la calcitonine et la ferritine à l'intestin grêle et/ou au côlon d'un mammifère qui comprend les étapes de :
A) préparation d'une série de mini-comprimés, chacun des mini-comprimés ayant une taille des particules inférieure à environ 5 mm et consistant essentiellement en un coeur de calcitonine ou de ferritine;
B) enrobage des mini-comprimés au moyen de deux membranes séparées et caractérisées de manière distincte, l'une des membranes consistant essentiellement en un polymère dépendant du pH qui est sensiblement soluble à un pH supérieur à environ 5,0 de sorte qu'il reste intact dans l'estomac et la partie finale de l'intestin, choisi parmi les copolymères anioniques à base d'acide méthacrylique et d'ester méthylique ou éthylique d'acide méthacrylique, d'acétophtalate de cellulose, de phtalate d'hydroxypropylméthylcellulose, d'acétophtalate de polyvinyle, de gommelaque, d'acétosuccinate d'hydroxypropylméthylcellulose, de carboxyméthylcellulose, d'acétotriméllitate de cellulose, des copolymères d'acide maléique et des dérivés d'acide phtalique et la deuxième des membranes consistant essentiellement en un ou plusieurs polymères choisis parmi les copolymères formés à partir des esters des acides acrylique et méthacrylique avec une faible teneur en groupes ammonium quaternaire, les copolymères neutres à base d'acrylate d'éthyle et de méthacrylate de méthyle et ayant un poids moléculaire moyen de 800 000, l'éthylcellulose, le polyéthylène, les polysiloxanes et leurs mélanges de sorte que la deuxième membrane soit sensiblement insoluble, mais perméable aux fluides gastro-intestinaux, de manière à ce qu'elle reste intacte afin de retarder la dissolution du médicament jusqu'à un moment qui peut varier de 30 minutes à 8 heures, et
C) si souhaité, formulation des mini-comprimés enrobés en des formes dosées médicamenteuses à administration orale, choisies parmi les capsules ou les comprimés.

2. Procédé de préparation d'une composition médicamenteuse à libération ciblée suivant la revendication 1, dans lequel la composition est caractérisée par la libération de pas plus d'environ 10% du médicament dans l'estomac, sensiblement tout le reste du médicament étant libéré dans l'intestin grêle et/ou le côlon en une période de 1/2 heure à 8 heures.

3. Procédé de préparation d'une formulation médicamenteuse ciblée suivant la revendication 1, dans lequel la composition est caractérisée par la libération de pas plus d'environ 10% du médicament à un pH inférieur à 4,5, sensiblement tout le reste du médicament étant libéré à un pH de 5,0 ou davantage, en une période d'environ 1/2 heure à 8 heures dans des milieux gastro-intestinaux simulés.

4. Procédé de préparation d'une composition suivant l'une quelconque des revendications 1 à 3, dans lequel la membrane contenant le polymère dépendant du pH est intérieure à l'autre membrane.

5. Procédé de préparation d'une composition suivant l'une quelconque des revendications 1 à 3, dans lequel la membrane contenant le polymère dépendant du pH est extérieure à l'autre membrane.

6. Procédé de préparation d'une composition suivant l'une quelconque des revendications 1 à 5, comprenant en outre, un polymère soluble dans l'eau, indépendant du pH, choisi parmi l'hydroxypropylméthylcellulose, l'hydroxypropylcellulose, l'hydroxyéthylcellulose, la méthylcellulose, la polyvinylpyrrolidone et leurs mélanges.

7. Procédé de préparation d'une composition suivant l'une quelconque des revendications précédentes, comprenant en outre, un plastifiant dans au moins l'une des membranes.

8. Procédé de préparation d'une composition suivant l'une quelconque des revendications 1 à 5, dans lequel la membrane sensiblement insoluble est choisie parmi l'éthylcellulose et les mélanges d'éthylcellulose avec l'hydroxypropylméthylcellulose, en un rapport de 1:5 à 5:1.

9. Procédé de préparation d'une composition suivant l'une quelconque des revendications 1 à 8, dans lequel les mini-comprimés ont une taille des particules située entre 0,1 mm et 5,0 mm.

10. Procédé de préparation d'une formulation médicamenteuse à libération ciblée suivant les revendications précédentes, où les mini-comprimés enrobés sont, en outre, formulés en des capsules ou des comprimés.

11. Procédé suivant l'une quelconque des revendications précédentes, dans lequel la formulation préparée est caractérisée par la libération de pas plus d'environ 11% après 3 heures, et de pas plus d'environ 75% après 6 heures, dans des sucs gastro-intestinaux simulés.

12. Procédé suivant l'une quelconque des revendications 1 à 9, dans lequel la composition préparée est caractérisée par la libération d'environ 90% du médicament en une période de 1 heure à 1 1/2 heure à pH 6,8.
